# EUROPEAN PATENT SPECIFICATION

(11) **EP 3 749 957 B1**
(45) Date of publication and mention of the grant of the patent: **19.06.2024**
(21) Application number: 19705933.0
(22) Date of filing: 07.02.2019
(51) Int. Cl.: G01N 33/577, G01N 33/68

(54) **ELASTIN ASSAY**
ELASTINTEST
DOSAGE DE L'ÉLASTINE

(30) Priority: 08.02.2018 GB 201802070
(43) Date of publication of application: 16.12.2020
(73) Proprietor: Nordic Bioscience A/S, 2730 Herlev (DK)
(72) Inventor: GUDMANN, Karoline, Natasja, Stæhr, 2700 Brønshøj (DK); SAND, Jannie, Marie, Bülow, 2760 Måløv (DK); MANON-JENSEN, Tina, 2605 Brøndby (DK); ØRSNES-LEEMING, Diana, 2730 Herlev (DK); KARSDAL, Morten, 2100 København Ø (DK)
(74) Representative: Beck Greener LLP
(86) International application number: PCT/EP2019/053003
(87) International publication number: WO 2019/154909

(56) References cited:
- EP-A2- 2 734 542
- WO-A1-2017/134172
- US-A1- 2016 223 568
- HE JIANGTAO ET AL: "Characterization of peptide fragments from lung elastin degradation in chronic obstructive pulmonary disease", EXPERIMENTAL LUNG RESE, INFORMA HEALTHCARE, vol. 36, no. 9, 1 November 2010 (2010-11-01), pages 548-557, XP009183777, ISSN: 1521-0499, DOI: 10.3109/01902148.2010.489143

## Description

### Field of the invention

The present invention relates to an elastin assay and its use in evaluating fibrotic diseases, such as COPD.

### Background

Chronic obstructive lung disease (COPD) is characterized by inflammation in the airways and an excessive concentration of neutrophils and mast cells (1,2). Neutrophils and mast cells synthesise and store preformed serine proteinases like cathepsin G (CG) and proteinase 3 (PR3) in granules from which they are released in response to pro-inflammatory mediators. Together these proteinases have a broad spectrum of activity against the extracellular matrix (ECM) (3,4). Elastic fibers are a major insoluble component of the extracellular matrix of the lungs, and are essential for structure, function, resilience and elasticity of the extracellular matrix. The elastic fibers are comprised by an inner core of cross-linked elastin embedded within fibrillin microfibrils (5). They create a thin and highly branched network throughout the respiratory tree to support the expansion and recoil of the alveoli during breathing. They are characterized by a high stability, and low turnover in healthy adult tissue. Only a few proteases, such as serine proteinases in addition to selected MMPs, are able to cleave elastin fibers by damaging the elastin core and microfibrils (6-8). In a normal inflammatory response the protease-antiprotease balance is maintained through the secretion of endogenous protease inhibitors (e.g. heparan sulfate) (9). In pathological conditions this balance is skewed, which leads to a loss of elasticity, which is a major pathological feature in COPD and emphysema (10,11). It has been suggested that both the serine proteinases CG and PR3 are upregulated in COPD, but there are currently no markers that are able to link increased degradation of elastin with those proteinases.

The present inventors have now developed an immunoassay for monitoring PR3 degradation of elastin, and have demonstrated its use in evaluating fibrotic diseases, in particular COPD.

### Summary of the Invention

Accordingly, in a first aspect the present invention relates to an immunoassay method for quantifying peptides having a C-terminus amino acid sequence LPGGYGLPYT (SEQ ID NO: 1) in a patient biofluid sample, said method comprising contacting said patient biofluid sample with a monoclonal antibody specifically reactive with said C-terminus amino acid sequence LPGGYGLPYT (SEQ ID NO: 1), and determining the amount of binding between said monoclonal antibody and said C-terminus amino acid sequence.

Preferably, the monoclonal antibody does not specifically recognise or bind a C-extended elongated version of said C-terminus amino acid sequence or a C-truncated shortened version of said C-terminus amino acid sequence. In this regard "C-extended elongated version of said C-terminus amino acid sequence" means one or more amino acids extending beyond the C-terminus of the sequence LPGGYGLPYT-COOH (SEQ ID NO: 1). For example, if the C-terminal amino acid sequence LPGGYGLPYT-COOH (SEQ ID NO: 1) was elongated by a threonine residue then the corresponding "C-extended elongated version" would be LPGGYGLPYTT-COOH (SEQ ID NO: 2). Similarly, "C-truncated shortened version of said C-terminus amino acid sequence" means one or more amino acids removed from the C-terminus of the sequence LPGGYGLPYT-COOH (SEQ ID NO: 1). For example, if the C-terminal amino acid sequence LPGGYGLPYT-COOH (SEQ ID NO: 1) was shortened by one amino acid residue then the corresponding "C-truncated shortened version" would be LPGGYGLPY-COOH (SEQ ID NO: 3).

The patient biofluid sample may be, but is not limited to, blood, urine, synovial fluid, serum, BALE (bronchoalveolar lavage fluid), or plasma.

The immunoassay may be, but is not limited to, a competition assay or a sandwich assay. Similarly, the immunoassay may be, but is not limited to, an enzyme-linked immunosorbent assay or a radioimmunoassay.

In a second aspect, the present invention relates to a method of immunoassay for detecting or quantifying a fibrotic disease in a patient, the method comprising contacting a patient biofluid sample with a monoclonal antibody specifically reactive with a C-terminus amino acid sequence LPGGYGLPYT (SEQ ID NO: 1), determining the amount of binding between said monoclonal antibody and peptides comprising said C-terminus amino acid sequence, and correlating said amount of binding with i) values associated with normal healthy subjects and/or ii) values associated with known fibrotic disease severity and/or iii) values obtained from said patient at a previous time point wherein a decrease in the amount of binding is indicative of a reduction in severity of the disease and/or iv) a predetermined cut-off value of at least 150.0 ng/ml being indicative of fibrosis.

Preferably, the fibrotic disease is chronic obstructive pulmonary disease (COPD).

The predetermined cut-off value is at least 150.0 ng/mL, more preferably at least 175.0 ng/mL, even more preferably at least 200.0 ng/mL, even more preferably at least 225.0 ng/mL, and most preferably at least 250 ng/mL. In this regard, through the combined use of various statistical analyses it has been found that a measured amount of binding between the monoclonal antibody (described above) and the C-terminus biomarker of at least 150.0 ng/mL or greater may be determinative of the presence of fibrosis, such as COPD. By having a statistical cutoff value of at least 150.0 ng/mL, more preferably at least 175.0 ng/mL, even more preferably at least 200.0 ng/mL, even more preferably at least 225.0 ng/mL, and most preferably at least 250.0 ng/mL, it is possible to utilise the method of the invention to diagnose fibrosis, such as COPD, with a high level of confidence. Or, in other words, applying the statistical cutoff value to the method of the invention is particularly advantageous as it results in a standalone diagnostic assay; i.e. it removes the need for any direct comparisons with healthy individuals and/or patients with known disease severity in order to arrive at a diagnostic conclusion. This may also be particularly advantageous when utilising the assay to evaluate patients that already have medical signs or symptoms that are generally indicative of fibrosis, such as COPD, (e.g. as determined by a physical examination and/or consultation with a medical professional) as it may act as a quick and definitive tool for corroborating the initial prognosis and thus potentially remove the need for more invasive procedures, such as endoscopy or biopsy, and expedite the commencement of a suitable treatment regimen. An expedited conclusive diagnosis may result in the disease being detected, and thus treated, at an earlier stage. Preferably, the predetermined cut-off value corresponds to the cut-off value as measured in human blood, serum or plasma.

Preferably, the monoclonal antibody does not specifically recognise or bind a C-extended elongated version of said C-terminus amino acid sequence or a C-truncated shortened version of said C-terminus amino acid sequence. The terms "C-extended" and "C-truncated" are explained supra.

The patient biofluid sample may be, but is not limited to, blood, urine, synovial fluid, serum, BALF, or plasma.

Also described herein but not forming part of the invention is a method for determining whether a patient is responding positively to a treatment for a fibrotic disease, such as COPD, wherein said method comprises using the method described supra to quantify the amount of peptides comprising the C-terminus amino acid sequence LPGGYGLPYT in at least two biological samples, said biological samples having been obtained from said patient at a first time point and at at least one subsequent time point during a period of administration of the treatment to said patient, and wherein a reduction in the quantity of peptides comprising the C-terminus amino acid sequence LPGGYGLPYT from said first time point to said at least one subsequent time point during the period of treatment is indicative of said patient responding positively to said treatment.

The above method may also be used to determine the efficacy of a novel therapeutic for treating a fibrotic disease, such as COPD. In that regard, a novel therapeutic will be considered efficacious if the quantity of peptides comprising the C-terminus amino acid sequence LPGGYGLPYT (SEQ ID NO: 1) is reduced from the said first time point to said at least one subsequent time point during the period of treatment using said novel therapeutic.

In a further aspect, the present invention relates to a monoclonal antibody specifically reactive with a C-terminus amino acid sequence LPGGYGLPYT (SEQ ID NO: 1). The term "monoclonal antibody" as used herein extends to intact monoclonal antibodies and antibody fragments thereof, such as F_{ab}, Fᵥ, etc., that are specifically reactive with a C-terminus amino acid sequence LPGGYGLPYT (SEQ ID NO: 1).

In another aspect, the present invention relates to an assay kit comprising a monoclonal antibody specifically reactive with a C-terminus amino acid sequence LPGGYGLPYT (SEQ ID NO: 1), and at least one of:
- a streptavidin coated well plate
- a biotinylated peptide Biotin-L-LPGGYGLPYT (SEQ ID NO: 5), wherein L is an optional linker
- a secondary antibody for use in a sandwich immunoassay
- a calibrator peptide comprising the C-terminus amino acid sequence LPGGYGLPYT (SEQ ID NO: 1)
- an antibody biotinylation kit
- an antibody HRP labeling kit
- an antibody radiolabeling kit

Preferably, the monoclonal antibody of the invention is raised against a synthetic peptide having the amino acid sequence LPGGYGLPYT (SEQ ID NO: 1).

### Definitions

As used herein the term "C-terminus" refers to the extremity of a polypeptide, i.e. at the C-terminal end of the polypeptide, and is not to be construed as meaning in the general direction thereof.

As used herein the term "competitive ELISA" refers to a competitive enzyme-linked immunosorbent assay and is a technique known to the person skilled in the art.

As used herein the term "sandwich immunoassay" refers to the use of at least two antibodies for the detection of an antigen in a sample, and is a technique known to the person skilled in the art.

As used herein the term "amount of binding" refers to the quantification of binding between antibody and biomarker, which said quantification is determined by comparing the measured values of biomarker in the biofluid samples against a calibration curve, wherein the calibration curve is produced using standard samples of known concentration of the biomarker. In the specific assay disclosed herein which measures in biofluids the C-terminus biomarker having the C-terminus amino acid sequence LPGGYGLPYT (SEQ ID NO: 1), the calibration curve is produced using standard samples of known concentration of the calibration peptide LPGGYGLPYT (SEQ ID NO: 1). The values measured in the biofluid samples are compared to the calibration curve to determine the actual quantity of biomarker in the sample. The present invention utilises spectrophotometric analysis to both produce the standard curve and measure the amount of binding in the biofluid samples; in the Examples set out below the method utilises HRP and TMB to produce a measurable colour intensity which is proportional to the amount of binding and which can be read by the spectrophotometer. Of course, any other suitable analytical method could also be used.

As used herein the "cut-off value" means an amount of binding that is determined statistically to be indicative of a high likelihood of a fibrotic disease, such as COPD, in a patient, in that a measured value of biomarker in a patient sample that is at or above the statistical cut-off value corresponds to at least a 70% probability, preferably at least an 80% probability, preferably at least an 85% probability, more preferably at least a 90% probability, and most preferably at least a 95% probability of the presence or likelihood of a fibrotic disease, such as COPD.

As used herein the term "values associated with normal healthy subjects and/or values associated with known disease severity" means standardised quantities of ELP-3 determined by the method described supra for subjects considered to be healthy, i.e. without a fibrotic disease, such as COPD, and/or standardised quantities of ELP-3 determined by the method described supra for subjects known to have a fibrotic disease, such as COPD, of a known severity.

As used herein, "ELP-3" refers to peptides having the C-terminus amino acid sequence LPGGYGLPYT (SEQ ID NO: 1), and "ELP-3 ELISA" refers to the specific competitive ELISA disclosed herein which quantifies in a sample the amount peptides having the C-terminus amino acid sequence LPGGYGLPYT (SEQ ID NO: 1).

### Figures

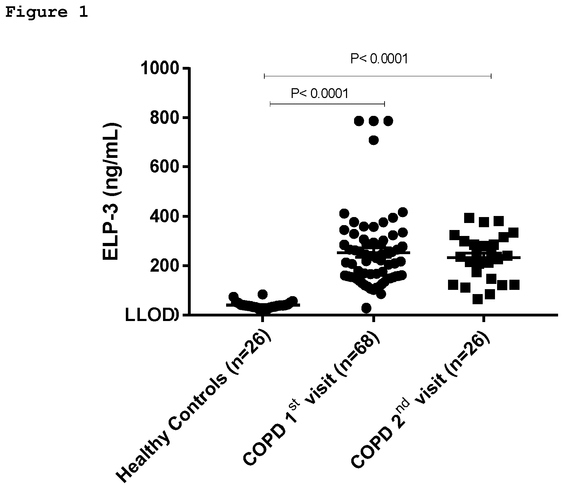

**Figure 1****.** Measurement of ELP-3 in healthy subjects and in patients with COPD.

### Examples

The presently disclosed embodiments are described in the following Examples, which are set forth to aid in the understanding of the disclosure, and should not be construed to limit in any way the scope of the disclosure as defined in the claims which follow thereafter. The following examples are put forth so as to provide those of ordinary skill in the art with a complete disclosure and description of how to make and use the described embodiments, and are not intended to limit the scope of the present disclosure nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

In the following examples, the following materials and methods were employed.

### Reagents

All applied reagents used in the presented experiments were high quality chemicals from Sigma Aldrich (St. Louis, Mo, USA) and Merck (Whitehouse Station, NJ, USA). The 96-well streptavidin-coated microtiterplates used were from Roche, Basel, Switzerland. The assay buffer applied consisted of 50mM Tris-buffered saline (TBS) 2g NaCl, pH 8.0. 3,3',5,5' - Tetramethylbenzidine (TMB) was from Kem-EN-Tec Diagnostics. The synthetic peptides used for immunization and assay development were 1) Immunogenic peptide: KLH-CGG-LPGGYGLPYT (SEQ ID NO: 4), Biotinylated peptide: Biotin-LPGGYGLPYT (SEQ ID NO: 5), 3) Standard peptide: LPGGYGLPYT (SEQ ID NO: 1), and 4) Elongated peptide: LPGGYGLPYTT (SEQ ID NO: 2). All synthetic peptides were purchased from American peptide Company, Sunnyvale, California USA. Reagents applied for in vitro cleavages: Elastin (Sigma, E7152) Human neutrophil elastase (HNE) protein (Abcam, ab91099), PR3 (EPC ML734), and complete protease inhibitor (Roche 1186153001).

### Selection of target sequence

Cleavage sites specific for PR3 in elastin (Uniprot: P15502 - ELN_Human) have previously been identified by mass spectrometry (12). The PR3 generated elastin decapeptides were blasted for homology to decapeptide sequences from other proteins using the NPS@: network protein sequence analysis. Based on this the sequence LPGGYGLPYT (SEQ ID NO: 1) was selected for the ELP-3 assay antibody development. The presence of the identified sequence was validated by mass spectrometry in-house analysis of PR3 cleavage of elastin in vitro.

### Antibody development

Immunizations were performed in 6 mice, for each selected target sequence, that were 4-6 weeks old using Freund's incomplete adjuvant (KLH-CGG-LPGGYGLPYT (SEQ ID NO: 4)) subcutaneously in the abdomen with 200ul emulsified antigen (50ug per immunization). The four initial immunizations were performed every second week followed by additional four immunizations performed every fourth week. The mouse with the highest serum titer was selected for fusion. The mouse was rested for a month and then boosted intravenously with 50ul immunogenic peptide in 100 ul 0.9% NaCl solution three days before isolation of the spleen. The spleen cells were fused with SP2/o myeloma cells to produce hybridroma as described by Gefter et al and cloned in culture dishes (13). The clones were plated into 96-well microtiter plates for further growth employing the limited dilution method to ensure monoclonal growth. Supernatants of antibody-producing hybridoma were screened for reactivity against standard peptide and native material in an indirect ELISA using streptavidin-coated plates. Biotin-LPGGYGLPYT (SEQ ID NO: 5) was used as screening peptide, while standard peptide LPGGYGLPYT (SEQ ID NO: 1) was used as a calibrator to test for further specificity of clones.

### ELISA protocol

Supernatant from a selected clone for the targeted sequence was collected and monoclonal antibody was purified using HiTrap affinity columns (GE Healthcare Life Science, Little Chalfront, Buckinghamshire, UK).

Based on the collected antibodies the ELP-3 assay was developed as competitive ELISA assay. A 96-well streptavidin-coated microtiter plate (Roche, Basel, Switzerland) was coated with 100ul biotinylated screening peptide (Biotin-LPGGYGLPYT (SEQ ID NO: 5)) dissolved in assay buffer for 30 min at 20°C with shaking. Plates were washed five times in washing buffer (20 mM TRIS, 50 mM NaCl, pH 7). Sample/standard/control (20ul) was added and followed by addition of (100ul) monoclonal antibody and incubated for 3h at 4°C with shaking. After incubation, plates were washed five times in washing buffer. A volume of 100 ul of secondary HRP labeled goat anti-mouse antibody was added followed by 1h incubation at 20°C with shaking. Plates were then washed and TMB was added and the plate was analyzed by a SpectraMax M reader (Molecular Devices, Ca, USA) at 450 nm with 650nm as the reference. A standard curve was plotted using a 4-parametric mathematical fit model. Each ELISA plate included kit controls to monitor inter-assay variation. All samples were measured within the range of the assay. All samples below the level of lower limit of quantification (LLOQ) were assigned the value of LLOQ.

### Technical evaluation

To determine linearity of dilution healthy human serum (n=4), heparin plasma (n=4), citrate plasma (n=4) and EDTA plasma was applied. Antibody specificity was calculated as percentage of signal inhibition of 2-fold diluted standard peptide (LPGGYGLPYT (SEQ ID NO: 1)), elongated peptide (LPGGYGLPYTT (SEQ ID NO: 2)), non-sense peptide (PGGVPGGVFY (SEQ ID NO: 6)) and non-sense coater (LPGGYGLPYT-K-Biotin (SEQ ID NO: 7)). The linarites were assessed by the percentage recovery of undiluted samples. The intra and inter-assay variation was determined by 10 independent measurements of 7 quality control samples run in double determinations. Lower limit of detection (LLOD) was determined as mean +3x standard deviations (SD) of 21 blank (buffer) samples. Upper limit of detection (ULOD) was determined as the mean - 3x SD measurements of standard A. Lower limit of quantification (LLOQ) was determined as the lowest concentration measured in human serum with an error lower than 30%. The accuracy was measured in healthy human serum samples spiked with standard peptide and in serum, and then calculated as the percentage recovery of spiked peptide or serum in buffer. The assay was tested for interference by calculating the percentage recovery of analyte in non-spiked healthy serum compared to human serum spiked with lipids (low=4.83mM, high= 10.98mM), hemoglobin (low=0.155mM, high=0.310mM), and biotin (low =30ng/ml, high =90ng/ml).

### Analyte stability

Analyte stability was evaluated by incubation of three healthy human serum and plasma samples at either 4 or 20°C for 2, 4 and 24 hours and calculated as the percentage of the samples kept at -20C (0 hour sample). Additionally, the freeze thaw stability was determined for three healthy human serum and plasma samples to up to four freeze and thaw.

### Cleavage analysis

In order to generate the ELP-3 fragment in vitro, elastin was incubated for 2, 4, 24 or 48 hours at 37°C with PR3 or HNE. Elastin was reconstituted in buffer (Tris-HCl, 150mM NaCl, pH 7.5) for a concentration of 1mg/ml. Cleavage solutions contained a final concentration of 100ug/ml elastin and 1ug/ml protease for the PR3 and 100ug/ml elastin and 2ug/ml protease for the HNE cleavage (buffer: Tris-HCl, 150mM NaCl, pH 7.5). All solutions were immediately frozen until analysis.

### Ethics statement

The work performed in mice was approved by the National Authority (The Animal Experiments inspectorate, approval number: 2013-15-2934-00956). All mice were treated according to the guidelines for animal welfare.

The assessment of ELP-3 in COPD patients was performed in a cohort previously described by Sand et al. (14). The study included 68 participants and complies with the Declaration of Helsinki and Good clinical practice Guidelines, was approved by the local ethics committee (protocol number H-6-2013-014). All participants provided informed consent before all study-related assessments.

Inclusion criteria were a COPD diagnosis and FEV1 <80% of predicted value. Exclusion criterion was an acute exacerbation of COPD leading to hospitalization within four weeks prior to the study. ELP-3 levels in the COPD patients were compared to the levels of commercially available control sera from healthy donors (Valley Bio-Medial, Winchester, VA, USA). All measurements were performed blinded.

### Statistical analyses

Differences in ELP-3 levels between healthy controls and COPD patients were assed using a two-tailed Mann-Whitney unpaired t-test. Comparision of age in healthy controls vs COPD patients was performed using the Mann- Whitney unpaired t-test. Differences were considered statistically significant if p<0.05. Asterisks indicate the following: *p<0.05, **p<0.01, ***p<0.001, ****p<0.0001. Graphs are shown as Mean ± Standard error of the mean. All statistical analyses were performed in GraphPad Prism v. 7 (Graph Pad Software, La Jolla, CA, USA) or MedCalc (Ostend, Belgium).

### Results

### Clone characterization

Supernatants from antibody producing hybridomas were screened for reactivity against standard peptide, elongated peptide, and truncated peptide in an indirect ELISA. The peptide Biotin-LPGGYGLPYT was used to screen for reactivity. The antibody clone NBH116/6A10-E7-D12 was selected for ELP-3 and antibodies were purified from the supernatant. Based on this the competitive ELISA assay was established. Native reactivity was observed in serum and plasma, and no inhibition was observed using the elongated peptide or non-sense peptide.

### Technical evaluation

The measurement was determined based on the linear range of the standard curve. The mean intra- and inter-assay variation ranged from 2.4-10% and 4.7-14.5%, respectively.

Linearity of dilution was observed when diluted 1+3 for human serum and citrate plasma (but not EDTA and heparin plasma). Serum samples were spiked with peptide or another serum sample in seven different concentrations. The spiking recovery for both serum and peptides were within an acceptable range. Mean recovery % values were within ±20% in reference with expected concentration of spiked samples and therefore acceptable. Analyte stability was acceptable during short term storage (up to 48 hours) at 4 and 20°C, during freeze/cycles and neither hemoglobin, lipids or biotin reduced the signal for any of the two assays.

### In vitro analysis - ELP-3 is generated by PR3

Elastin was incubated with different elastases in order to determine whether ELP-3 was specific for proteinase 3 cleavage. ELP-3 was generated by elastin cleaved by PR3 after 2-48 h. Detectable levels of fragments recognized by ELP-3 were generated by HNE cleavage, but in much lower concentrations compared to the respective assay. No cross reactivity was observed towards the intact elastin, buffer control or protease controls for the assay.

### ELP-3 levels were increased in COPD as compared to healthy controls

To determine whether ELP-3 levels are increased in COPD, ELP-3 was assessed in a previously described cohort of 68 patients with clinically stable COPD. Of these, 26 also attended a follow-up visit after four weeks. Twenty two samples from healthy donors were included for comparison. Demographics can be seen in Table 1 and have previously been described by Sand et al. (14).

**Table 1. Patient demographics. Mean (SD)**

| | **COPD (n=68)** | **Healthy control (n=26)** | **p-value** |
|---|---|---|---|
| Age, Mean (SD) | 71.1 (9.0) | | <0.0001 |
| Male, n (%) | 40 (59) | | 0.52 |
| BMI, Mean (SD) | 24.5 (6.1) | NA | - |
| FEV1 % of predicted value | 40.4 (16.3) | NA | - |
| FEV/FVC ratio% | 49.6 (0.14) | NA | - |
| GOLD 2/3/4, % | 34%/34%/32% | NA | - |
| ELP-3 (ng/ml), Median (95% CI) | 217.9 (168.6-255.0) | 38.4 (33.8-42.3) | p<0.0001 |

As demonstrated in Figure 1, ELP-3 is statistically elevated in COPD patients when compared to healthy subjects (p<0.0001).

In this specification, unless expressly otherwise indicated, the word 'or' is used in the sense of an operator that returns a true value when either or both of the stated conditions is met, as opposed to the operator `exclusive or' which requires that only one of the conditions is met. The word 'comprising' is used in the sense of 'including' rather than in to mean 'consisting of'.

### References

1. Barnes et al., Nat Rev Dis Prim, 2015: 15076
2. Decramer et al., Lancet, 2012, 379:1341-1351
3. Owen, Int J Chron Obstruct Pulmon Dis, 2008, 3:253-268
4. Barnes et al., Pharmacol Rev, 2004, 56:515
5. Baldwin et al., Expert Rev Mol Med, 2013, 15:e8
6. Kielty et al., FEES Lett, 1994, 351:85-89
7. Ashworth et al., Biochem J, 1999, 340 (Pt 1:171-181)
8. Petersen et al., Eur J Vase Endovasc Surg, 2001, 22:48-52
9. Craciun et al., Glycobiology, 2016, 26:701-709
10. Dillon et al., Am Rev Respir Dis, 1992, 146:1143-1148
11. Schriver et al., Am Rev Respir Dis, 1992, 145:762-766
12. Heinz et al., Biochimie, 2012, 94:192-202
13. Gefter et al., Somatic Cell Genet, 1977, 3:231-236
14. Sand et al., Respir Res, 2015, 16:69

## Claims

1. An immunoassay method for quantifying peptides having a C-terminus amino acid sequence LPGGYGLPYT (SEQ ID NO: 1) in a patient biofluid sample, said method comprising contacting said patient biofluid sample with a monoclonal antibody specifically reactive with said C-terminus amino acid sequence LPGGYGLPYT (SEQ ID NO: 1), and determining the amount of binding between said monoclonal antibody and said C-terminus amino acid sequence.

2. The method of claim 1, wherein the monoclonal antibody does not specifically recognise or bind a C-extended elongated version of said C-terminus amino acid sequence or a C-truncated shortened version of said C-terminus amino acid sequence.

3. The method of claims 1 or 2, wherein the patient biofluid sample is blood, urine, synovial fluid, serum, BALF, or plasma.

4. A method of immunoassay for detecting or quantifying a fibrotic disease in a patient, the method comprising contacting a patient biofluid sample with a monoclonal antibody specifically reactive with a C-terminus amino acid sequence LPGGYGLPYT (SEQ ID NO: 1), determining the amount of binding between said monoclonal antibody and peptides comprising said C-terminus amino acid sequence, and correlating said amount of binding with i) values associated with normal healthy subjects and/or ii) values associated with known fibrotic disease severity and/or iii) values obtained from said patient at a previous time point wherein a decrease in the amount of binding is indicative of a reduction in severity of the disease and/or iv) a predetermined cut-off value of at least 150.0 ng/ml being indicative of fibrosis.

5. A method as claimed in claim 4, wherein the fibrotic disease is chronic obstructive pulmonary disease (COPD).

6. The method of claims 4 or 5, wherein the monoclonal antibody does not specifically recognise or bind a C-extended elongated version of said C-terminus amino acid sequence or a C-truncated shortened version of said C-terminus amino acid sequence.

7. The method of claims 4 to 6, wherein the patient biofluid sample is blood, urine, synovial fluid, serum, BALF, or plasma.

8. A monoclonal antibody specifically reactive with a C-terminus amino acid sequence LPGGYGLPYT (SEQ ID NO: 1).

9. An assay kit comprising a monoclonal antibody specifically reactive with a C-terminus amino acid sequence LPGGYGLPYT (SEQ ID NO: 1), and at least one of:
- a streptavidin coated well plate
- a biotinylated peptide Biotin-L-LPGGYGLPYT (SEQ ID NO: 5), wherein L is an optional linker
- a secondary antibody for use in a sandwich immunoassay
- a calibrator peptide comprising the C-terminus amino acid sequence LPGGYGLPYT (SEQ ID NO: 1)
- an antibody biotinylation kit
- an antibody HRP labeling kit
- an antibody radiolabeling kit

10. An assay kit as claimed in claim 9, wherein the monoclonal antibody is raised against a synthetic peptide having the amino acid sequence LPGGYGLPYT (SEQ ID NO: 1).

## Patentansprüche

1. Immunoassay-Verfahren zum Quantifizieren von Peptiden mit einer C-terminalen Aminosäuresequenz LPGGYGLPYT (SEQ ID NO: 1) in einer Patienten-Biofluidprobe, wobei das Verfahren Zusammenbringen der Patienten-Biofluidprobe mit einem monoklonalen Antikörper, der spezifisch mit der C-terminalen Aminosäuresequenz LPGGYGLPYT (SEQ ID NO: 1) reaktiv ist, und Bestimmen der Bindungsmenge zwischen dem monoklonalen Antikörper und der C-terminalen Aminosäuresequenz umfasst.

2. Verfahren nach Anspruch 1, wobei der monoklonale Antikörper eine C-verlängerte längere Version der C-terminalen Aminosäuresequenz oder eine C-verkürzte kürzere Version der C-terminalen Aminosäuresequenz nicht spezifisch erkennt oder bindet.

3. Verfahren nach Anspruch 1 oder 2, wobei die Biofluidprobe des Patienten Blut, Urin, Synovialfluid, Serum, BALF oder Plasma ist.

4. Immunoassay-Verfahren zum Nachweisen oder Quantifizieren einer fibrotischen Erkrankung bei einem Patienten, wobei das Verfahren Zusammenbringen einer Patienten-Biofluidprobe mit einem monoklonalen Antikörper, der spezifisch mit einer C-terminalen Aminosäuresequenz LPGGYGLPYT (SEQ ID NO: 1) reagiert, Bestimmen der Bindungsmenge zwischen dem monoklonalen Antikörper und Peptiden, die die C-terminale Aminosäuresequenz umfassen, und Korrelieren der Bindungsmenge mit i) Werten, die normalen gesunden Probanden zugeordnet sind, und/oder ii) Werten, die dem Schweregrad einer bekannten fibrotischen Erkrankung zugeordnet sind, und/oder iii) Werten, die von dem Patienten zu einem früheren Zeitpunkt erlangt wurden, wobei eine Abnahme der Bindungsmenge eine Verringerung des Schweregrads der Erkrankung angibt und/oder iv) ein vorbestimmter Testtrennwert von mindestens 150,0 ng/ml eine Fibrose angibt.

5. Verfahren nach Anspruch 4, wobei die fibrotische Erkrankung eine chronisch obstruktive Lungenerkrankung (COPD) ist.

6. Verfahren nach Anspruch 4 oder 5, wobei der monoklonale Antikörper eine C-verlängerte längere Version der C-terminalen Aminosäuresequenz oder eine C-verkürzte kürzere Version der C-terminalen Aminosäuresequenz nicht spezifisch erkennt oder bindet.

7. Verfahren nach einem der Ansprüche 4 bis 6, wobei die Patienten-Biofluidprobe Blut, Urin, Synovialfluid, Serum, BALF oder Plasma ist.

8. Monoklonaler Antikörper, der spezifisch mit einer C-terminalen Aminosäuresequenz LPGGYGLPYT (SEQ ID NO: 1) reaktiv ist.

9. Assay-Kit, umfassend einen monoklonalen Antikörper, der spezifisch mit einer C-terminalen Aminosäuresequenz LPGGYGLPYT (SEQ ID NO: 1) reaktiv ist, und mindestens eines von Folgendem:
- einer mit Streptavidin beschichteten Platte mit Vertiefungen
- einer biotinylierten Peptid Biotin-L-LPGGYGLPYT (SEQ ID NO: 5), wobei L ein optionaler Linker ist,
- einem sekundären Antikörper zur Verwendung in einem Sandwich-Immunoassay
- einem Kalibrierungspeptid, das die C-terminale Aminosäuresequenz LPGGYGLPYT (SEQ ID NO: 1) umfasst,
- einem Antikörper-Biotinylierungs-Kit
- einem Antikörper-HRP-Markierungs-Kit
- einem Antikörper-Radioaktivmarkierungs-Kit

10. Assay-Kit nach Anspruch 9, wobei der monoklonale Antikörper gegen ein synthetisches Peptid mit der Aminosäuresequenz LPGGYGLPYT (SEQ ID NO: 1) gezüchtet wird.

## Revendications

1. Procédé de dosage immunologique pour quantifier des peptides ayant une séquence d'acides aminés C-terminale LPGGYGLPYT (SEQ ID N° : 1) dans un échantillon de fluide biologique de patient, ledit procédé comprenant la mise en contact dudit échantillon de fluide biologique de patient avec un anticorps monoclonal spécifiquement réactif avec ladite séquence d'acides aminés C-terminale LPGGYGLPYT (SEQ ID N° : 1), et la détermination du degré de liaison entre ledit anticorps monoclonal et ladite séquence d'acides aminés C-terminale.

2. Procédé selon la revendication 1, dans lequel l'anticorps monoclonal ne reconnaît pas spécifiquement ni ne se lie à une version allongée, étendue à l'extrémité C, de ladite séquence d'acides aminés C-terminale ou une version raccourcie, tronquée à l'extrémité C, de ladite séquence d'acides aminés C-terminale.

3. Procédé selon les revendications 1 ou 2, dans lequel l'échantillon de fluide biologique de patient est du sang, de l'urine, du liquide synovial, du sérum, du fluide BAL ou du plasma.

4. Procédé de dosage immunologique pour détecter ou quantifier une maladie fibrotique chez un patient, le procédé comprenant la mise en contact d'un échantillon de fluide biologique de patient avec un anticorps monoclonal spécifiquement réactif avec une séquence d'acides aminés C-terminale LPGGYGLPYT (SEQ ID N° : 1), la détermination du degré de liaison entre ledit anticorps monoclonal et les peptides comprenant ladite séquence d'acides aminés C-terminale, et la corrélation dudit degré de liaison avec i) des valeurs associées à des sujets sains normaux et/ou ii) des valeurs associées à une gravité connue de maladie fibrotique et/ou iii) des valeurs obtenues dudit patient à un moment antérieur où une diminution du degré de liaison indique une réduction de la gravité de la maladie et/ou iv) une valeur seuil prédéterminée d'au moins 150,0 ng/ml étant indicative de fibrose.

5. Procédé selon la revendication 4, dans lequel la maladie fibrotique est une maladie pulmonaire obstructive chronique (BPCO).

6. Procédé selon les revendications 4 ou 5, dans lequel l'anticorps monoclonal ne reconnaît pas spécifiquement ni ne se lie à une version allongée, étendue à l'extrémité C, de ladite séquence d'acides aminés C-terminale ou une version raccourcie, tronquée à l'extrémité C, de ladite séquence d'acides aminés C-terminale.

7. Procédé selon les revendications 4 à 6, dans lequel l'échantillon de fluide biologique de patient est du sang, de l'urine, du liquide synovial, du sérum, du fluide BAL ou du plasma.

8. Anticorps monoclonal spécifiquement réactif avec une séquence d'acides aminés C-terminale LPGGYGLPYT (SEQ ID N° : 1).

9. Trousse de dosage comprenant un anticorps monoclonal spécifiquement réactif avec une séquence d'acides aminés C-terminale LPGGYGLPYT (SEQ ID N° : 1), et au moins l'un parmi :
- une plaque à puits revêtue de streptavidine
- un peptide biotinylé Biotin-L-LPGGYGLPYT (SEQ ID N° : 5), où L est un lieur facultatif
- un anticorps secondaire pour une utilisation dans un dosage sandwich
- un peptide étalon comprenant la séquence d'acides aminés C-terminale LPGGYGLPYT (SEQ ID N° : 1)
- un kit de biotinylation d'anticorps
- un kit de marquage HRP d'anticorps
- un kit de radiomarquage d'anticorps.

10. Trousse de dosage selon la revendication 9, dans laquelle l'anticorps monoclonal est dirigé contre un peptide synthétique ayant la séquence d'acides aminés LPGGYGLPYT (SEQ ID N° : 1).
